# EUROPEAN PATENT SPECIFICATION

(11) **EP 3 706 710 B1**
(45) Date of publication and mention of the grant of the patent: **10.04.2024**
(21) Application number: 18807130.2
(22) Date of filing: 08.11.2018
(51) Int. Cl.: A61K 9/00, A61K 47/10, A61K 45/06, A61P 19/02, A61K 31/443, A61K 31/4465, A61K 8/22

(54) **EXTENDED RELEASE FORMULATIONS FOR INTRA-ARTICULAR APPLICATIONS**
FORMULIERUNGEN MIT VERLÄNGERTER FREISETZUNG FÜR INTRAARTIKULÄRE ANWENDUNGEN
FORMULATIONS À LIBÉRATION PROLONGÉE POUR APPLICATIONS INTRA-ARTICULAIRES

(30) Priority: 10.11.2017 US 201762584589 P; 10.10.2018 US 201862743864 P
(43) Date of publication of application: 16.09.2020
(73) Proprietor: Novartis AG, 4056 Basel (CH)
(72) Inventor: CASTAGNOLI, Carlo, 4002 Basel (CH); FISCH, Andreas, 4002 Basel (CH); LORSCHEIDER, Mathilde, 4056 Basel (CH); NEHARKAR, Manjali Laxman, Hyderabad 500 0181 (IN); RIEBESEHL, Bernd Ulrich, 4002 Basel (CH)
(74) Representative: Brennan, Méabh Bridget
(86) International application number: PCT/IB2018/058788
(87) International publication number: WO 2019/092637

(56) References cited:
- WO-A1-2012/172072
- US-A1- 2017 239 227
- D Ramya Devi ET AL: "Poloxamer: A Novel Functional Molecule For Drug Delivery And Gene Therapy", Journal of Pharmaceutical Sciences and Research, 1 August 2013 (2013-08-01), page 159, XP55546338, Cuddalore Retrieved from the Internet: URL:https://pdfs.semanticscholar.org/3252/ bcbb2763f63d3ff603ffd9dfecf6d2bcf0a1.pdf [retrieved on 2019-01-23]

## Description

### CROSS-REFERENCE TO RELATED APPLICATIONS

This application claims the benefit of U.S. provisional application serial no. 62/584,589 filed 10 November 2017 and of U.S provisional application serial no. 62/743,864 filed 10 October 2018.

### FIELD OF THE INVENTION

The present invention relates to extended release formulations for treating or preventing joint damage resulting from arthritis, joint injury or cartilage injury.

The invention is defined in the appended claims. References to methods of treatment in this description are to be interpreted as references to the pharmaceutical compositions, combinations and medicaments of the present invention for use in a method of treatment of the human (or animal) body by therapy (or for diagnosis).

### BACKGROUND OF THE INVENTION

Arthritis is the inflammation of one of more joints, and affects approximately 350 million people worldwide. Osteoarthritis (OA) is the most common form of arthritis, and is characterized by a slow degenerative breakdown of the joint including both the articular cartilage and the subchondral bone underlying the articular cartilage. Joint damage (e.g., acute joint injury, such as a meniscal or ligament tear, or an intra-articular fracture) can also lead to arthritis, e.g., post-traumatic arthritis. Because articular cartilage has a limited ability to repair, even small undetectable damage can often get worse over time and lead to OA.

PCT/US15/30303 describes N-(3,4-dichlorophenyl)-3-(pyridin-4-yl)-7-oxabicyclo[2.2.1] heptane-2-carboxamide (Compound A) and other compounds that are useful in preventing, ameliorating or treating arthritis and/or joint injury.

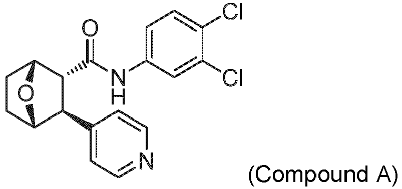

Compound A is weakly basic with a pKa of 5.2, and is poorly soluble in neutral pH and basic pH aqueous solutions, but exhibits a strong pH dependent solubility. Solubility increases with decreasing pH. Compound A can be formulated as immediate release formulations for intra-articular injection. However, due to a high fluid exchange between the synovial fluid and the blood stream, immediate release formulations have a short synovial residence half-life and require frequent injections.

Thus, there remains a need for formulations that maintain efficacious levels of the drug substance in the synovial space for as long as possible, for treating chronic indications.

### SUMMARY OF THE INVENTION

The present invention provides extended release formulations comprising N-(3,4-dichlorophenyl)-3-(pyridin-4-yl)-7-oxabicyclo[2.2.1] heptane-2-carboxamide or a pharmaceutically acceptable salt thereof.

In one aspect, the present invention provides a pharmaceutical composition comprising an aqueous suspension of: (i) microcrystalline N-(3,4-dichlorophenyl)-3-(pyridin-4-yl)-7-oxabicyclo[2.2.1]heptane-2-carboxamide or a pharmaceutically acceptable salt thereof; and (ii) a surfactant comprising a water-soluble co-polymer characterized by > 5% solubility in water at 25 °C.

As used herein, % concentrations of ingredients in the compositions are in w/v %, unless otherwise stated.

In one embodiment, the composition is an injectable extended release formulation.

In one embodiment, the pharmaceutical composition as described herein, wherein said surfactant is a water-soluble block co-polymer of formula 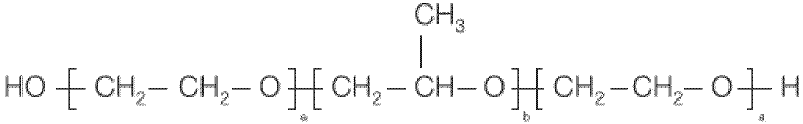 wherein:
a is 75-101; and
b is 25-60.

In one embodiment, the pharmaceutical composition as described herein, wherein said water-soluble block co-polymer is poloxamer 188.

In one embodiment, the pharmaceutical composition as described herein, wherein said water-soluble block co-polymer is poloxamer 407.

In one embodiment, the pharmaceutical composition as described herein, wherein the concentration of said water-soluble block co-polymer is at least 0.025 % w/v; for example, between 0.025-2% w/v, between 0.025-1% w/v, or preferably between 0.05-1% w/v.

In one embodiment, the pharmaceutical composition as described herein, wherein said surfactant further comprises sodium lauryl sulfate. In one embodiment, the concentration of said sodium lauryl sulfate is at least 0.05% w/v; more particularly, between 0.05-0.5 % w/v.

In one embodiment, the pharmaceutical composition as described herein, further comprising a suspension stabilizer. In one embodiment, the concentration of said suspension stabilizer is between 0.1-10% w/v.

In one embodiment, the pharmaceutical composition as described herein, wherein said suspension stabilizer is selected from: (i) polyvinylpyrrolidone having an average molecular weight between 1-10 kDa, and optionally between 2-5 kDa; (ii) carboxymethyl cellulose having an average molecular weight between 25-2500 kDa, and optionally between 75-125 kDa; and (iii) a combination thereof.

In one embodiment, the pharmaceutical composition as described herein, wherein the concentration of said polyvinylpyrrolidone is between 1-5% w/v, and optionally between about 2-4% w/v; and the concentration of said carboxymethyl cellulose is 0.5-2% (w/v), and optionally between 0.75-1.5% w/v.

In one embodiment, the pharmaceutical composition as described herein wherein said polyvinylpyrrolidone is PVP-12.

In one embodiment, the pharmaceutical composition as described herein, wherein said aqueous suspension comprises: (i) between 1-400 mg of microcrystalline N-(3,4-dichlorophenyl)-3-(pyridin-4-yl)-7-oxabicyclo[2.2.1] heptane-2-carboxamide per 1 mL of said aqueous suspension; (ii) between 0.05-1% w/v of water-soluble block co-polymer, optionally wherein said water-soluble block co-polymer is poloxamer 188 or poloxamer 407; and (iii) between 1-5% w/v of polyvinylpyrrolidone, optionally wherein said polyvinylpyrrolidone is PVP-K12.

In one embodiment, the pharmaceutical composition as described herein, comprising between 10-30 mg or about 25 mg of microcrystalline N-(3,4-dichlorophenyl)-3-(pyridin-4-yl)-7-oxabicyclo[2.2.1]heptane-2-carboxamide per 1 mL of said aqueous suspension.

In one embodiment, the pharmaceutical composition as described herein, comprising between 0.05-0.15% w/v, 0.08-0.12% w/v, or about 0.1% w/v of poloxamer 407.

In one embodiment, the pharmaceutical composition as described herein, comprising up to 75 mg, optionally between 40-60 mg or about 50 mg of microcrystalline N-(3,4-dichlorophenyl)-3-(pyridin-4-yl)-7-oxabicyclo[2.2.1]heptane-2-carboxamide per 1 mL of said aqueous suspension.

In one embodiment,. the pharmaceutical composition as described herein, comprising between 80-120 mg or about 100 mg of microcrystalline N-(3,4-dichlorophenyl)-3-(pyridin-4-yl)-7-oxabicyclo[2.2.1]heptane-2-carboxamide per 1 mL of said aqueous suspension.

In one embodiment, the pharmaceutical composition as described herein, comprising between 0.1-0.3% w/v or about 0.2% w/v poloxamer 407.

In one embodiment, the pharmaceutical composition as described herein, comprising between 150-250 mg or about 200 mg of microcrystalline N-(3,4-dichlorophenyl)-3-(pyridin-4-yl)-7-oxabicyclo[2.2.1]heptane-2-carboxamide per 1 mL of said aqueous suspension.

In one embodiment, the pharmaceutical composition as described herein, comprising between 0.1-0.5% w/v or about 0.2-0.4% w/v poloxamer 407.

In one embodiment, the pharmaceutical composition as described herein comprising between 350-450 mg or about 400 mg of microcrystalline N-(3,4-dichlorophenyl)-3-(pyridin-4-yl)-7-oxabicyclo[2.2.1]heptane-2-carboxamide per 1 mL of said aqueous suspension.

In one embodiment, the pharmaceutical composition as described herein, comprising between 0.6-1% w/v, 0.7-0.9% w/v, or about 0.8% w/v poloxamer 407.

In one embodiment, the pharmaceutical composition as described herein, comprising between 1.5-2.5% w/v, or about 2% w/v PVP-K12.

In one embodiment, the pharmaceutical composition as described herein, comprising between 1-5% w/v, or about 2-4% w/v PVP-K12.

In one embodiment, the pharmaceutical composition as described herein, wherein said aqueous suspension comprises: (i) between 1-100 mg of microcrystalline N-(3,4-dichlorophenyl)-3-(pyridin-4-yl)-7-oxabicyclo[2.2.1]heptane-2-carboxamide per 1 mL of said aqueous suspension; (ii) between 0.5-1.5 mg or about 1 mg poloxamer 407 per 1 mL of said aqueous suspension; and (iii) between 15-25 mg or about 20 mg PVP-K12 per 1 mL of said aqueous suspension.

In one embodiment, the pharmaceutical composition as described herein, comprising between 20-30 mg or about 25 mg of microcrystalline N-(3,4-dichlorophenyl)-3-(pyridin-4-yl)-7-oxabicyclo[2.2.1]heptane-2-carboxamide per 1 mL of said aqueous suspension.

In one embodiment, the pharmaceutical composition as described herein, wherein said crystalline N-(3,4-dichlorophenyl)-3-(pyridin-4-yl)-7-oxabicyclo[2.2.1]heptane-2-carboxamide is microcrystalline or micronized.

In one embodiment, he pharmaceutical composition as described herein, wherein said microcrystalline N-(3,4-dichlorophenyl)-3-(pyridin-4-yl)-7-oxabicyclo[2.2.1]heptane-2-carboxamide has a particle size distribution D90 of 50 microns or less, by laser light diffraction in suspension at a wavelength between 610-650 nm, and more particularly at about 630-635 nm.

In one embodiment, the pharmaceutical composition as described herein, wherein said microcrystalline N-(3,4-dichlorophenyl)-3-(pyridin-4-yl)-7-oxabicyclo[2.2.1]heptane-2-carboxamide has a particle size distribution distribution D90 of 25 microns or less, by laser light diffraction in suspension, at a wavelength between 610-650 nm, and more particularly at about 630-635 nm.

In one embodiment, the pharmaceutical composition as described herein, wherein said N-(3,4-dichlorophenyl)-3-(pyridin-4-yl)-7-oxabicyclo[2.2.1]heptane-2-carboxamide is further defined as (1R,2R,3S,4S)-N-(3,4-dichlorophenyl)-3-(pyridin-4-yl)-7-oxabicyclo[2.2.1] heptane-2-carboxamide.

In one embodiment, the pharmaceutical composition as described herein, further comprising a buffer capable of maintaining the pH of the suspension between 6 and 8.

In one embodiment, the pharmaceutical composition as described herein, wherein said composition is suitable for intra-articular injection.

In one embodiment, the pharmaceutical composition as described herein, wherein said composition is suitable for intra-articular injection into a synovial cavity, particularly of the knee joint, in a patient suffering from arthritis, joint injury or cartilage injury. In one embodiment, the composition is suitable for intra-articular injection into the synovial cavity of a patient suffering from osteoarthritis. In another embodiment, the composition is suitable for intra-articular injection into the synovial cavity of a patient suffering from trauma arthritis. In another embodiment, the composition is suitable for intra-articular injection into the synovial cavity of a patient suffering from autoimmune arthritis.

In one embodiment, the pharmaceutical composition as described herein, wherein said composition provides extended release of N-(3,4-dichlorophenyl)-3-(pyridin-4-yl)-7-oxabicyclo[2.2.1]heptane-2-carboxamide into the synovial cavity for more than one hour, 24 hours, 7 days, 14 days or 30 days.

In one embodiment, the pharmaceutical composition as described herein, wherein said composition is suitable for administration with a 22-31 gauge needle, a 29-31 gauge needle, or preferably a 30-gauge needle.

In another embodiment, there is provided a combination comprising the pharmaceutical composition as described herein, and a second therapeutic agent.

In another embodiment, there is provided a pharmaceutical composition as described herein, and optionally in combination with a second therapeutic agent, for treating, ameliorating or preventing joint damage or injury, such as arthritis (osteoarthritis, trauma arthritis, or autoimmune arthritis such as systemic rheumatoid arthritis); degenerative disc disease; acute joint injury or cartilage injury.

In another embodiment, there is provided a pharmaceutical composition as described herein and optionally in combination with a second therapeutic agent, for inducing hyaline cartilage production, or for inducing differentiation of chondrocytes.

In another embodiment, there is provided the use of a pharmaceutical composition as described herein, and optionally in combination with a second therapeutic agent, for the manufacture of a medicament for the treatment of joint damage or injury, such as arthritis (osteoarthritis, trauma arthritis, or autoimmune arthritis such as systemic rheumatoid arthritis); degenerative disc disease; acute joint injury or cartilage injury.

In another embodiment, there is provided the use of a pharmaceutical composition as described herein, and optionally in combination with a second therapeutic agent, for the manufacture of a medicament for inducing hyaline cartilage production, or for inducing differentiation of chondrocytes.

In another embodiment, there is provided a pharmaceutical composition as described herein for use in a method for treating, ameliorating or preventing acute join damage or injury in a subject in need thereof, wherein said pharmaceutical composition is administered to said subject in a dose range of up to 25 mg, up to 75 mg, up to 100 mg, up to 200 mg or up to 400 mg, of microcrystalline N-(3,4-dichlorophenyl)-3-(pyridin-4-yl)-7-oxabicyclo[2.2.1]heptane-2-carboxamide.

In a further embodiment, there is provided a pharmaceutical composition as described herein for use in a methodcomprising injecting the pharmaceutical composition into a synovial cavity of an individual having osteoarthritis.

In a further embodiment, there is provided a pharmaceutical composition as described herein for use in a method of inducing hyaline cartilage production or differentiation of chondrocytes, comprising contacting chondrogenic progenitor cells with a therapeutically effective amount of the pharmaceutical composition as described herein, and optionally in combination with a second therapeutic agent; thereby inducing producing hyaline cartilage extracellular matrix.

In a further embodiment, there is provided a pharmaceutical composition as described herein for use in a method of inducing hyaline cartilage production or differentiation of chondrocytes as described herein, wherein said contacting step is performed *in vitro* or *in vivo* in a mammal; and when *in vivo,* stem cells are present in the mammal.

In a further embodiment, there is provided a pharmaceutical composition as described herein for use in a method of inducing hyaline cartilage production or differentiation of chondrocytes as described herein, wherein said contacting step occurs in a matrix or biocompatible scaffold.

In a further embodiment, there are provided the pharmaceutical composition as described herein, the use as described herein, wherein said second therapeutic agent is selected from angiopoietin-like 3 protein (ANGPTL3), insulin growth factor (IGF1), SM04690, Janus kinase inhibitor, oral salmon calcitonin, SD-6010, vitamin D3, collagen hydrolysate, bone morphogenetic protein 7 (BMP7), rusalatide acetate, avocado soy unsaponifiables (ASU), a steroid, a non-steroidal anti-inflammatory agent (NSAID), hyaluronic acid, kartogenin, and TPX-100.

### BRIEF DESCRIPTION OF THE DRAWINGS

Figure 1 (FIG. 1) is an electron microscope image of Compound A after three months at 40 °C.
Figure 2 (FIG. 2) compares the rat plasma concentration of Compound A after intra-articular administration of three extended release formulations of Compound A: microcrystal suspension, PLGA microparticle suspension, and MLV liposome suspension.
Figure 3 (FIG. 3) compares the *in vivo* profile of a microcrystal extended release suspension of Compound A (250 µg) with an immediate release solution formulation (91 ng).

### DETAILED DESCRIPTION OF THE INVENTION

The present invention provides extended release formulations comprising a microcrystal suspension of N-(3,4-dichlorophenyl)-3-(pyridin-4-yl)-7-oxabicyclo[2.2.1] heptane-2-carboxamide or a pharmaceutically acceptable salt thereof, and a surfactant comprising a water-soluble co-polymer.

The present invention overcomes multiple challenges for preparing a microcrystal suspension of Compound A, including the identification of excipients that stabilize the drug suspension and provides acceptable features during manufacturing (compounding/ filling) and administration (dosing accuracy, syringeability) of a highly concentrated crystal suspension (up to 100 mg/mL), including but not limited to the following:
- Aspects for compounding:
   ∘ Quick suspendability of the drug substance (DS) (good wettability of the DS)
   ∘ Slow sedimentation of the initial suspension (prevention of aggregation of the DS microcrystals) in order to assure dosing precision during Good Manufacturing Practice (GMP) manufacturing
- Aspects after autoclaving or storage:
   ∘ Chemical stability of the drug substance;
   ∘ Quick re-suspendability of micro-crystals
   ∘ Syringeability through 30 gauge needles to allow pain-less injection in the knee
   ∘ Slow sedimentation to allow dosing precision
   ∘ Absence of big crystals (Oswald ripening) or aggregates

### Definitions

As used herein, "extended release" refers to a dosage form that is deliberately modified to protract the release rate of the drug substance compared to that observed for an immediate-release dosage form. The release pattern in an extended release dosage may begin with a burst effect that mimics an immediate release, followed by a slower release of the remaining drug substance in the dosage form.

As used herein, the term "about" means within a statistically meaningful range of a value, typically within 10%. Such a range can lie within experimental error, typical of standard methods used for the measurement and/or determination of a given value or range. In one embodiment, the range is within 5% of the indicated value. In another embodiment, the range is within 1% of the indicated value. In yet another embodiment, the range is within 0.5% of the indicated value.

As used herein, the term "subject" refers to primates (e.g., humans, male or female), dogs, rabbits, guinea pigs, pigs, rats, mice and horses. In certain embodiments, the subject is a primate. In yet other embodiments, the subject is a human.

As used herein, the term "treat", "treating" or "treatment" of any disease or disorder refers to alleviating or ameliorating the disease or disorder (i.e., slowing or arresting the development of the disease or at least one of the clinical symptoms thereof); or alleviating or ameliorating at least one physical parameter or biomarker associated with the disease or disorder, including those which may not be discernible to the patient.

As used herein, the term "prevent", "preventing" or "prevention" of any disease or disorder refers to the prophylactic treatment of the disease or disorder; or delaying the onset or progression of the disease or disorder

As used herein, a subject is "in need of" a treatment if such subject would benefit biologically, medically or in quality of life from such treatment.

As used herein, the term "a therapeutically effective amount" of a pharmaceutical composition refers to an amount of the composition that will elicit the biological or medical response of a subject, for example, reduction or inhibition of an enzyme or a protein activity, or ameliorate symptoms, alleviate conditions, slow or delay disease progression, or prevent a disease, etc. In one non-limiting embodiment, the term "a therapeutically effective amount" refers to an amount of the Compound A extended release formulation that, when administered to a subject, is effective to (1) at least partially alleviate, inhibit, prevent and/or ameliorate joint damage resulting from joint injury and arthritis. In another non-limiting embodiment, the term "a therapeutically effective amount" refers to an amount of the Compound A extended release formulation that, when administered to a cell, or a tissue, or a non-cellular biological material, or a medium, is effective to promote chondrogenesis.

As used herein, the terms "treat", "treating", "treatment" plus "ameliorate" and "ameliorating" refer to any indicia of success in the treatment or amelioration of an injury, pathology, condition, or symptom (e.g., pain), including any objective or subjective parameter such as abatement; remission; diminishing of symptoms or making the symptom, injury, pathology or condition more tolerable to the patient; decreasing the frequency or duration of the symptom or condition; or, in some situations, preventing the onset of the symptom or condition. The treatment or amelioration of symptoms can be based on any objective or subjective parameter; including, e.g., the result of a physical examination.

As used herein, "administering" refers to administration to a specific joint.

As used herein, the term "pharmaceutical composition" refers to a compound or a pharmaceutically acceptable salt thereof, together with at least one pharmaceutically acceptable carrier, in a form suitable for oral or parenteral administration.

As used herein, the term "pharmaceutically acceptable carrier" refers to a substance useful in the preparation or use of a pharmaceutical composition and includes, for example, suitable diluents, solvents, dispersion media, surfactants, antioxidants, preservatives, isotonic agents, buffering agents, emulsifiers, absorption delaying agents, salts, drug stabilizers, binders, excipients, disintegration agents, lubricants, wetting agents, sweetening agents, flavoring agents, dyes, and combinations thereof, as would be known to those skilled in the art (see, for example, Remington The Science and Practice of Pharmacy, 22nd Ed. Pharmaceutical Press, 2013, pp. 1049-1070).

As used herein, the term "a," "an," "the", "said" and similar terms used in the context of the present invention (especially in the context of the claims) are to be construed to cover both the singular and plural unless otherwise indicated herein or clearly contradicted by the context.

The present invention provides extended release formulations comprising N-(3,4-dichlorophenyl)-3-(pyridin-4-yl)-7-oxabicyclo[2.2.1] heptane-2-carboxamide or a pharmaceutically acceptable salt thereof, particularly an injectable extended release formulation suitable for intra-articular injection to a joint of a patient suffering from arthritis, joint injury or cartilage injury.

In one aspect, the present invention provides a pharmaceutical composition comprising an aqueous suspension of: (i) microcrystalline N-(3,4-dichlorophenyl)-3-(pyridin-4-yl)-7-oxabicyclo[2.2.1]heptane-2-carboxamide or a pharmaceutically acceptable salt thereof; and (ii) a surfactant comprising a water-soluble co-polymer characterized by > 5% solubility in water at 25 °C.

The hydrophilic-lipophilic balance (HLB) of a surfactant is a measure of the degree to which it is hydrophilic or lipophilic, and is determined by calculating values for the different regions of the molecule using methods known to those skilled in the art (e.g., BASF^{®} PLURACARE^{®} UF Grades Poloxamer Technical Information, 04_070801e-01 July 2009; BASF^{®} Kolliphor^{®} P Grades Technical Information, 03_111136e-03).

Examples of water-soluble block co-polymers, which may be suitable for use with the pharmaceutical compositions disclosed herein include but are not limited to poloxamer 188 (LUTROL^{®} F-68), poloxamer 237 (PLURONIC^{®} F-87), poloxamer 338 (PLURONIC^{®} F-108) and poloxamer 407 (PLURONIC^{®} F-127 or LUTROL^{®} F-127), or a mixture thereof. The concentration of the poloxamer is generally about ≥ 0.025 %; for example, between 0.025-2% w/v.

In another embodiment, the pharmaceutical composition may further comprise a suspension stabilizer, such as polyvinylpyrrolidone (PVP), carboxymethyl cellulose or a combination thereof. In particular embodiments, the additional suspension stabilizer is PVP-K12, alone or in combination with carboxymethyl cellulose.

In yet another embodiment, the pharmaceutical composition further comprises a suitable buffer capable of maintaining the pH of the aqueous suspension at physiologically acceptable pH between 6-8 or about 7.2-7.4. In one embodiment, the pharmaceutical composition comprises a phosphate buffer. Other known buffering agents, including but are not limited to, organic acid salts, TRIS or tromethamine hydrochloride, may be considered for use with the pharmaceutical compositions disclosed herein. In another embodiment, the pharmaceutical composition comprises NaCl for injection.

The pharmaceutical compositions of present invention may be administered simultaneously with, before or after, one or more other therapeutic agent(s). The pharmaceutical compositions of the present invention may be administered separately or together with one or more therapeutic agent, by the same or different routes of administration. A therapeutic agent is, for example, a chemical compound, peptide, antibody, antibody fragment or nucleic acid, which is therapeutically active or enhances the therapeutic activity when administered to a patient in combination with an extended release formulation of Compound A or a pharmaceutically salt thereof.

In one embodiment, the invention provides a product comprising an extended release formulation of Compound A or a pharmaceutically acceptable salt thereof, and at least one other therapeutic agent as a combined preparation for simultaneous, separate or sequential use in therapy. In one embodiment, the therapy is the treatment of joint damage resulting from joint injury or arthritis. Products provided as a combined preparation include a composition comprising an extended release formulation of Compound A or a pharmaceutically acceptable salt thereof, and the other therapeutic agent(s) together in the same pharmaceutical composition; or an extended release formulation of Compound A or a pharmaceutically acceptable salt thereof and the other therapeutic agent(s) in separate form, e.g. in the form of a kit.

In one embodiment, the invention provides an extended release formulation of Compound A or a pharmaceutically acceptable salt thereof in combination with a second therapeutic agent. The second agent may be one or more additional chondrocyte differentiation agent(s). Examples of chondrocyte differentiation agent include but are not limited to angiopoietin-like 3 protein (ANGPTL3), insulin growth factor (IGF1), SM04690 (Wnt inhibitor), Janus kinase inhibitors (such as ruxolitinib, tofacitinib, baricitinib), oral salmon calcitonin, SD-6010 (iNOS inhibitor), vitamin D3 (cholecalciferol), collagen hydrolyzate, bone morphogenetic protein 7 (BMP7), rusalatide acetate, avocado soy unsaponifiables (ASU), a steroid, a non-steroidal anti-inflammatory agent (NSAID), hyaluronic acid, kartogenin and TPX-100.

The pharmaceutical composition or combination of the present invention can be in a unit dosage of 0.5-1000 mg of active ingredient(s) for a subject of about 50-70 kg; for example, in a unit dosage range of 0.5-500 mg, 0.5-250 mg, 0.5-150 mg, 0.5-100 mg, or 0.5-50 mg of active ingredient(s). The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The above-cited dosage properties are demonstrable in *in vitro* and *in vivo* tests using advantageously mammals, *e.g.*, mice, rats, dogs, monkeys or isolated organs, tissues and preparations thereof. The compositions of the present invention can be applied *in vitro* in the form of solutions, *e.g.,* aqueous solutions, and *in vivo* either enterally, parenterally, advantageously intravenously, *e.g.,* as a suspension or in aqueous solution. The dosage *in vitro* may range between about 10⁻³ molar and 10⁻⁹ molar concentrations.

In one embodiment, the pharmaceutical composition of the present invention is administered intra-articularly in a dose range of up to 25 mg of active ingredient (e.g., Compound A); for example, about 0.5 mg, 2.5 mg, 7.5 mg, 15 mg or 25 mg of active ingredient. In another embodiment, the pharmaceutical composition of the present invention is administered intra-articularly in a dose range of up to 75 mg of active ingredient; for example, about 40 mg, 50 mg, 60 mg or 75 mg of active ingredient. In yet another embodiment, the pharmaceutical composition of the present invention is administered intra-articularly in a dose range of up to 100 mg of active ingredient; for example between 50-100 mg of active ingredient. In yet another embodiment, the pharmaceutical composition of the present invention is administered intra-articularly in a dose range of up to 200 mg or 400 mg of active ingredient.

In the combination therapies of the invention, the compositions of the invention and the other therapeutic agent may be manufactured and/or formulated by the same or different manufacturers. Moreover, the compound of the invention and the other therapeutic may be brought together into a combination therapy: (i) prior to release of the combination product to physicians (*e.g*. in the case of a kit comprising the compound of the invention and the other therapeutic agent); (ii) by the physician themselves (or under the guidance of the physician) shortly before administration; (iii) in the patient themselves, *e.g*. during sequential administration of the compound of the invention and the other therapeutic agent.

### EXAMPLES

The following examples exemplify the invention. One skilled in the art will understand that w/v % in the pharmaceutical compositions of the invention further includes acceptable variations within a statistically meaningful range and within acceptable experimental error, for example within 10% of the indicated value, and more preferably, within 5%, 1% or 0.5% of the indicated value.

### Abbreviations

The following abbreviations are used.
- PVP-K12: Polyvinylpyrrolidone (Povidone K12); M.W. 3500 (CAS 9003-39-8)
- CMC: Carboxymethyl cellulose sodium salt
- F68: LUTROL^{®} F-68 (BASF)
- F127: LUTROL^{®} F-127 (BASF)
- SDS: Sodium dodecyl sulfate (sodium lauryl sulfate)
- Egg PC: L-α-phosphatidylcholine (natural lipids); MW 770.123 (average); (CAS 9281-44-2; Avanti Polar Lipids)
- Phosphate buffer: 50 nM, pH 7.0 (2.88g of KH₂PO₄ and 4.099g of Na₂HPO₄ in 1L)
- WFI: Water for Injection

### ASSAYS

Several assays were designed to test whether the formulations meet the criteria for intra-articular administration.

Suspendability. This assay measures the ability of the formulation to wet Compound A microcrystals and maintain the particles in suspension. Microcrystal suspensions of Compound A were stirred continuously for 5, 15, 30, and 60 minutes. At the end of the stirring period, each solution was evaluated according to the following criteria: (i) whether a homogenous suspension has been achieved; (ii) whether drug particles adhere to the vial glass surface; (iii) whether homogenous suspension has been achieved, but with residual cake adhering to the bottom; or (iv) whether the drug substance formed large agglomerates.

Sedimentation. This assay measures the sedimentation rate of the initial suspension. A slow sedimentation indicates less aggregation of the drug particles, ensuring better dosing precision. After the stirring step above, sample formulations were allowed to settle for 5, 15, 30 and 60 minutes. At the end of each period, each sample was evaluated following the same criteria for suspendability.

Syringeability. This assay measures whether a formulation can be quickly delivered to a joint without causing pain to the patient. A homogenous suspension was pulled up using a 30 gauge syringe, and re-injected back to the same vial. Syringeability was subjectively determined based on ease of pushing out the suspension from the needle.

### Example 1. Preparation of Micro-crystals of Compound A

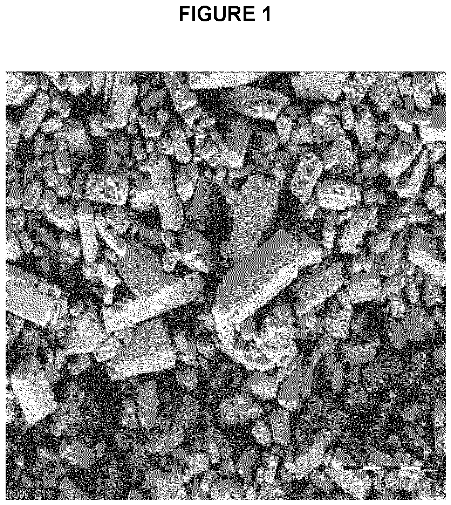

A production batch of Compound A was observed under the electronic microscope (FIG. 1). The particles were irregular and columnar, and ranged from about 100 nm up to 140 µm in length, but most of the particles are smaller than 50 µm. These particles have a tendency to agglomerate.

Compound A can be micronized using any methods known to those skilled in the art. Unmilled Compound A is subjected to a size reduction process to obtain particle size. The size reduction of Compound A can be performed using any known methods such as by jet-milling technology, and more particularly, by spiral jet-milling technology, fluidized bed opposed jet-milling technology or loop jet-milling technology.

In one embodiment, Compound A has a particle size distribution of D90 ≤ 50 µm. In another embodiment, Compound A has a particle size distribution of D90 ≤ 30 µm, ≤ 25 µm, ≤ 20 µm or ≤ 15 µm.

In yet other embodiments, Compound A was micronized to form micro-crystals with maximal particle diameters characterized in Table 1.

**Table 1**

| Parameter | Result |
|---|---|
| Surface area | 3 m²/g |
| Particle size: D10 | ≤1.4 µm |
| Particle size: D50 | ≤6.1 µm |
| Particle size: D90 | ≤ 29.7 µm |

### Example 2. Formulation Screening with PVP-K12 or CMC

Microcrystal suspensions of Compound A prepared in Example 1 were prepared with solutions containing PVP-K12 or carboxymethyl cellulose (CMC) sodium salt. The re-suspendability of the formulations was tested by repeating centrifugation and resuspension by shaking. The CMC containing formulations did not resuspend after centrifugation. PVP-K12 containing formulations were further investigated.

### Example 3. Formulations P1-P8 (PVP-K12 with F68, F127, SDS, or egg PC)

Placebo solutions P1 to P8 were prepared according to the composition (w/v %) listed in Table 2.

**Table 2**

| Formulation | PVP-K12 (%) | Additional excipient | Additional excipient (%) |
|---|---|---|---|
| P1 | 2% | F68 | 0.2% |
| P2 | 2% | F68 | 0.5% |
| P3 | 2% | F127 | 0.2% |
| P4 | 2% | F127 | 0.5% |
| P5 | 2% | SDS | 0.05% |
| P6 | 2% | SDS | 0.01% |
| P7 | 2% | Egg PC | 0.2% |
| P8 | 2% | Egg PC | 0.5% |

Formulations were prepared in R2 vials with about 5 mg of Compound A microcrystals, and filled with placebo solutions P1 to P8 to reach a drug substance concentration of 5 mg/mL. The formulations were tested for re-suspendability and sedimentation rate according to the sequential steps listed in Column 1 of Table 3. After each step, the turbidity of the samples was observed and documented by photographs, and summarized in Table 3.

**Table 3**

| | P1 | P2 | P3 | P4 | P5 | P6 | P7 | P8 |
|---|---|---|---|---|---|---|---|---|
| **Step** | | | | | | | | |
| Stirring over night | Turbid | Turbid | Turbid | Turbid | Turbid | Turbid | Turbid | Turbid |
| Sedimentation 1 h | Partially clear | Partially clear | Partially clear | Partially clear | Turbid | Turbid | Turbid | Turbid |
| Sedimentation 4 h | Clear | Clear | Partially clear | Partially clear | Turbid | Turbid | Turbid | Turbid |
| 30 Gauge Syringeability after 15 min. stirring | OK | OK | OK | OK | OK | OK | OK | OK |
| Centrifugation 1 h (2000 g) | Clear | Clear | Clear | Clear | Clear | Clear | Partially clear | Partially clear |
| Resuspension with hand shaking | Possible within 30 sec | Possible within 30 sec | Possible within 30 sec | Possible within 30 sec | Possible within 1 min | Possible within 1 min | Possible within 1 min | Possible within 1 min |
| Heat sterilization | Clear | Clear | Partially clear | Partially clear | Turbid | Turbid | Turbid | Turbid |
| Stirring over night | Turbid | Turbid | Turbid | Turbid | Turbid | Turbid | Turbid | Turbid |
| Sedimentation 1 h | Partially clear | Partially clear | Partially clear | Partially clear | Turbid | Turbid | Turbid | Turbid |
| Sedimentation 4h | Clear | clear | Partially clear | Partially clear | Turbid | Turbid | Turbid | Turbid |
| 30 G Syringeability | OK | OK | OK | OK | OK | OK | OK | OK |
| Centrifugation 1h (2000 g) | Clear | Clear | Clear | Clear | Clear | Clear | Partially clear | Partially clear |
| Resuspension with hand shaking | Possible within 30 sec | Possible within 30 sec | Possible within 30 sec | Possible within 30 sec | Possible within 1 min | Possible within 1 min | Possible within 1 min | Possible within 1 min |
| Light microscopy after 15 min. stirring | No large crystals >50 µm | No large crystals >50 µm | No large crystals >50 µm | No large crystals >50 µm | No large crystals >50 µm | No large crystals >50 µm | No large crystals >50 µm | No large crystals >50 µm |
| Resuspension after 4 h centrifugation 4000rpm (2000g) | Possible within 1 min | Possible within 1 min | Possible within 1 min | Possible within 1 min | Not possible within 3 min | Not possible within 3 min | Not possible within 3 min | Not possible within 3 min |
| Resuspension after 8 h centrifugation 4000rpm (2000g) | Possible within 3 min | Possible within 3 min | Possible within 5 min | Possible within 5 min | Not possible within 3 min | Not possible within 3 min | Not possible within 3 min | Not possible within 3 min |

The syringeability test provides a rough indication whether the formulation can be smoothly injected into a joint. For each vial, the suspension was withdrawn into a syringe through a 30 gauge needle and re-injected back to the same vial. The ease or difficulty of ejecting the content was categorized as "OK" or with difficulty.

Heat sterilization was performed at 122.5 °C and for 20 minutes. The suspendability and sedimentation of pre-heat sterilized and post-heat sterilized samples were observed to be the same. Thus, heat sterilization did not seem to have an effect on degradation and agglomeration of the microcrystals. Optical micrographs were taken from each resuspended formulations and screened for particles having an average particle size > 50 µm; and no particles having an average particle size ≥ 50 µm were found in all formulations. The distribution of the micronized crystals was homogenous in all cases (micrographs not shown).

Formulations comprising SDS and egg PC (P5-P8) exhibited poor re-suspendability after 4 hrs of centrifugation. On the other hand, formulations comprising F68 and F127 (P1-P4) exhibited better re-suspendability, and were further investigated.

### Example 4. Formulations P9-P16 (PVP-K12 with F68 or F127)

Placebo solutions P9 to P16 were prepared according to the composition (w/v %) listed in Table 4.

**Table 4**

| Formulation | PVP-K12 (%) | Additional excipient | Additional excipient (%) |
|---|---|---|---|
| P9 | 2% | F68 | 0.1% |
| P10 | 2% | F68 | 0.05% |
| P11 | 4% | F68 | 0.1% |
| P12 | 4% | F68 | 0.05% |
| P13 | 2% | F127 | 0.1% |
| P14 | 2% | F127 | 0.05% |
| P15 | 4% | F127 | 0.1% |
| P16 | 4% | F127 | 0.05% |

The formulations were prepared in R2 vials with about 5 mg of Compound A microcrystals added and filled up with placebo solutions P9 to P16 to reach a drug substance concentration of 5 mg/mL. The formulations were tested for re-suspendability and sedimentation rate according to the sequential steps listed in Column 1 of Table 5. After each step, the turbidity of the samples was observed and summarized in Table 5.

**Table 5**

| | P9 | P10 | P11 | P12 | P13 | P14 | P15 | P16 |
|---|---|---|---|---|---|---|---|---|
| **Step** | | | | | | | | |
| Stirring 1 h | turbid | turbid | turbid | turbid | turbid | turbid | turbid | turbid |
| 30 G Syringeability test | OK | OK | OK | OK | OK | OK | OK | OK |
| Resuspension after 4 h centrifugation 4000rpm (2000g) | Possible within 1 min | Possible within 1 min | Possible within 3 min | Possible within 3 min | Possible within 30 sec | Possible within 30 sec | Possible within 30 sec | Possible within 30 sec |
| 1 h stirring | Turbid | Turbid | Turbid | Turbid | Turbid | Turbid | Turbid | Turbid |
| 30 min sedimentation | Partially clear | Partially clear | Partially clear | Partially clear | Turbid | Partially turbid | Partially turbid | Partially turbid |
| 1 h sedimentation | Almost clear | Almost clear | Almost clear | Almost clear | Turbid | Partially clear | Partially clear | Partially clear |
| 2 h sedimentation | Clear | Clear | Clear | Clear | Turbid | Almost clear | Almost clear | Almost clear |
| 4 h sedimentation | Clear | Clear | Clear | Clear | Turbid | Clear | Clear | Clear |
| 19 h sedimentation | Clear | Clear | Clear | Clear | Partially clear | Clear | Clear | Clear |

Table 5 shows that all formulations containing F68 and F127 exhibited good resuspendability after a four-hour centrifugation. However, formulations containing F127 (P13-P16) were able to re-suspend the microcrystals within a shorter time and maintain the microcrystals in suspension longer when compared with formulations containing F68.

### Example 5. Formulations P17-P36 (PVP-K12 and F127)

Formulations P17 to P36, with or without F127, were designed as described in Table 6, with components listed as w/v %. As used herein, "Cpd A" refers to Compound A.

**Table 6**

| No. | PVP-K12 2% | F127 0.05% | SDS 0.01% | SDS 0.003% | SDS 0.001% | NaCl 0.875% | Mannitol 5.45% | Phosphate 5 mM pH 7.0 | Cpd A 5mg/mL |
|---|---|---|---|---|---|---|---|---|---|
| P17 | x | x | | | | x | | x | x |
| P18 | x | | x | | | x | | x | x |
| P18A | x | x | x | | | x | | x | x |
| P19 | x | | | x | | x | | x | x |
| P20 | x | | | | x | x | | x | x |
| P21 | x | | | | | x | | x | x |
| P22 | x | x | | | | | x | x | x |
| P23 | x | | x | | | | x | x | x |
| P23A | x | x | x | | | | x | x | x |
| P24 | x | | | x | | | x | x | x |
| P25 | x | | | | x | | x | x | x |
| P26 | x | | | | | | x | x | x |
| P27 | | x | | | | x | | x | x |
| P28 | | | x | | | x | | x | x |
| P28A | | x | x | | | x | | x | x |
| P29 | | | | x | | x | | x | x |
| P30 | | | | | x | x | | x | x |
| P31 | | | | | | x | | x | x |
| P32 | | x | | | | | x | x | x |
| P33 | | | x | | | | x | x | x |
| P33A | | x | x | | | | x | x | x |
| P34 | | | | x | | | x | x | x |
| P35 | | | | | x | | x | x | x |
| P36 | | | | | | | x | x | x |

The above formulations P17 to P36 were prepared from their individual placebo solutions by adding in the target amounts of the microcrystals. The placebo solutions (vehicles) were prepared from stock solutions of each component. The components (in w/v %) and volume (in mL) of the component solutions needed to prepare a 50 mL placebo solution for each of the formulations, and the pH of the placebo solutions, were listed in Table 7.

**Table 7**

| No. | PVP-K12 8.0% | F127 1.0% | SDS 0.1% | NaCl 17.5mg/mL | Mannitol 109mg/mL | Phosphate 5 mM pH 7.0 | WFI (mL) | pH Bulk Placebo |
|---|---|---|---|---|---|---|---|---|
| P17 | 12.5 | 2.5 | | 25 | | 5 | 5 | 6.8 |
| P18 | 12.5 | | 5 | 25 | | 5 | 2.5 | 6.79 |
| P18A | 12.5 | 2.5 | 5 | 25 | | 5 | 0 | 6.79 |
| P19 | 12.5 | | 1.5 | 25 | | 5 | 6 | 6.84 |
| P20 | 12.5 | | 0.5 | 25 | | 5 | 7 | 6.85 |
| P21 | 12.5 | | | 25 | | 5 | 7.5 | 6.87 |
| P22 | 12.5 | 2.5 | | | 25 | 5 | 5 | 7.14 |
| P23 | 12.5 | | 5 | | 25 | 5 | 2.5 | 7.07 |
| P23A | 12.5 | 2.5 | 5 | | 25 | 5 | 0 | 7.06 |
| P24 | 12.5 | | 1.5 | | 25 | 5 | 6 | 7.03 |
| P25 | 12.5 | | 0.5 | | 25 | 5 | 7 | 7.05 |
| P26 | 12.5 | | | | 25 | 5 | 7.5 | 7.15 |
| P27 | | 2.5 | | 25 | | 5 | 17.5 | 6.95 |
| P28 | | | 5 | 25 | | 5 | 15 | 6.98 |
| P28A | | 2.5 | 5 | 25 | | 5 | 12.5 | 6.97 |
| P29 | | | 1.5 | 25 | | 5 | 18.5 | 6.94 |
| P30 | | | 0.5 | 25 | | 5 | 19.5 | 6.89 |
| P31 | | | | 25 | | 5 | 20 | 6.85 |
| P32 | | 2.5 | | | 25 | 5 | 17.5 | 7.06 |
| P33 | | | 5 | | 25 | 5 | 15 | 7.2 |
| P33A | | 2.5 | 5 | | 25 | 5 | 12.5 | 7.21 |
| P34 | | | 1.5 | | 25 | 5 | 18.5 | 7.21 |
| P35 | | | 0.5 | | 25 | 5 | 19.5 | 7.22 |
| P36 | | | | | 25 | 5 | 20 | 7.13 |

Table 8 describes the preparation of stock solutions for each excipient.

**Table 8**

| | |
|---|---|
| PVP-K12 8% | For 200 mL: weighed 16 g of PVP-K12 in 200 mL volumetric flask and completed with water. |
| F127 1% | For 50 mL: weighed 0.5 g of F127 in 50 mL volumetric flask and completed with water. |
| SDS 0.1% (5/50) | For 50 mL: weighed 0.05 g of SDS in 50 mL volumetric flask and completed with water. |
| SDS 0.1% (1.5/50) | For 10 mL: weighed 0.01 g of SDS in10 mL volumetric flask and completed with water |
| SDS 0.1% (0.5/50) | For 5 mL: weighed 0.005 g of SDS in 5 mL volumetric flask and completed with water. |
| NaCl (17.5 mg/mL) | For 500 mL, weighed 8.75 g of NaCl in 500 mL volumetric flask and completed with water |
| Mannitol (109 mg/mL) | For 500 mL: weighed 54.5 g of Mannitol in 500 mL volumetric flask and completed with water |
| Phosphate buffer 50 mM pH 7.0 | For 1L: weighed 2.88 g of KH₂PO₄ and 4.1 g of Na₂HPO₄ in 1L volumetric flask and completed with water |

For each placebo solutions at 50 ml, the drug substance was added (5 mg/mL) and completed with the corresponding placebo by volume. Two vials of at least 1 mL of each formulation were prepared for subsequent assays. The first set of vials was assayed for their suspendability and sedimentation characteristics. The formulations were stirred continuously for 5, 15, 30, and 60 minutes. After 60 minutes of continued stirring, vials 17, 18, 18A, 19, 22, 23A, 27, 28 were homogeneous. The pH values measured at the completion of the stirring cycle compared well with those taken of the initial placebo solutions.

The formulations were tested under various assays to determine syringeability, sedimentation rate and suspendability. For syringeability, each sample formulation was withdrawn into a syringe through a 30 gauge needle and re-injected back to the same vial. All formulations had acceptable syringeability, even though the withdrawal of the samples was slow. Subsequently, the sample formulations were allowed to sediment for 60 minutes. After 60 minutes, vials 17, 18, 18A, 22, 23, 23A, 24, 27, 28A, 32 and 33A were still under suspension; vials 19, 20, 21, 25, 26 and 28 started to settle; and the rest of the vials had complete sedimentation. The vials were crimped shut and autoclaved at 122.5 °C for 20 minutes. All of the samples were clear, indicating that the microcrystals had sedimented. After hand shaking the vial a few times, only vials P17, P18, 23A, P27, and P28 re-suspended to homogenous suspensions. The second set of vials was subjected to five thermocycling cycles, each about 12 hours at 2-8 °C and 50 °C, and evaluated as described above. The samples were hand shaken to re-suspend the particles, only vials P17, 18, 19, 27 and 28 were resuspended. However, most formulations (but not all) resuspended after more vigorous shaking. Syringeability was acceptable for all vials, except for P20 and P22.

Another sedimentation analysis was conducted. After 60 minutes, vials P17, P18A, P22, P23, P23A, P24, P27, P28A, P32 and P33A were still under suspension. The samples were transferred to Eppendorf tubes and centrifuged at 4000 rpm for 4 hours. Subsequently, the samples were shaken to resuspend. More than half resuspended after some vigorous shaking (vortexing), but only vials P17, P22, P27, P32 and P33A consistently resuspended after 60 minutes and after centrifugation.

### Example 6. Exemplary Extended Release Formulation

Example 6 provides an exemplary composition to illustrate but not limit the invention. The pharmaceutical compositions of the invention further includes acceptable variations known to one of ordinary skill in the art.

| **Component** | **[mg/mL]** |
|---|---|
| Compound A microcrystals | Up to 100 |
| PVP-K12 | 20 |
| Poloxamer 407 (LUTROL ^{®} F127) | 1 |
| NaCl | 8.75 |
| Phosphate pH7 | 0.71 |

Surprisingly, the selected microcrystal suspension could be terminally sterilized by moist heat and did not show any signs of crystal growth (Oswald ripening) or aggregation during sterilization and over storage allowing accurate dosing through a thin needle (30G). No local tissue irritation were observed. In one embodiment, the composition comprises 25 mg/mL Compound A microcrystals as a uniform suspension in the buffered vehicle above.

### Example 7. Extended Release Formulations Comparative Data

Several formulations with extended release characteristics were prepared and tested. The highest possible drug substance load was used in each formulation to maximize the therapeutic effect.

### Microcrystal Suspensions

Compound A (250 µg) was suspended in 25 µL of buffered vehicle from Example 6.

### PLGA Microparticle Suspension

Compound A (2% w/v) and PLGA (12 kDa; 1:1 L:G ratio) in dichloromethane was emulsified in an aqueous solution containing 1% w/v polyvinyl alcohol (PVA) to stabilize the initial emulsion. Evaporation of dichloromethane by heat resulted in Compound A molecularly dispersed in PLGA microparticles, which were subsequently washed in water and dried. PLGA microparticles having a particle size distribution of D50=40 µm and D90=57 µm were obtained.

### Liposome Suspension

A liposome formulation was used to solubilize the Compound A in a lipid bilayer and to obtain a possible sustained release. Additionally, the liposome should help as a lubricant at the injected side. A liposome formula was prepared as described in the procedures below:
First procedure
   1. DMPC (100 mg) and unmicronized Compound A (3 mg) were dissolved in 10 mL EtOH:DCM 1:1
   2. EtOH and DCM were removed on the rotavap for 30 min at 40°C and 250 mbar (140 rpm)
   3. Traces of EtOH and DCM were removed by 10 min at 40°C and 40 mbar (140 rpm)
   4. Lipid/Compound A film were rehydrated with 1 mL phosphate-buffered saline (PBS) or sugars in water
   5. Samples where extruded through 1 um filter to obtain uniform sizes
   6. Samples were frozen at -20°C
   7. Samples were lyophilized overnight
   8. Samples were resuspended with sterile water
Second procedure
   1. Compound A and DMPC where dissolved in tert-butanol
   2. Samples were frozen at-20°C
   3. Samples were lyophilized overnight
   4. Samples were resuspended with PBS

Original materials could be sterile filtered by solubilizing DMPC and Compound A in tert-butanol. After lyophilization, the powder could be reconstituted to manufacture multilamellar vesicles (MLV). The used lipids (DMPC) in its manufactured form (MLV) have the advantages of potentially better retention at the side of injection due to its large size and anti-inflammatory properties. Leakage of Compound A was tested in a mouse model, where a fast release of Compound A was observed.

### Comparative Data

Compound A was dosed intra-articularly (IA) in different extended release formulations to male Lewis Rats, 3 animal per formulation. The PLGA microparticle and MLV liposome suspensions were administered near the maximal possible dose, which may be limited by drug-loading capacity. Plasma samples were serial collected post IA injections up to 480 hours (20 days, n=3 per timepoint).

Plasma concentrations of Compound A were quantified using a Liquid Chromatography/ Mass Spectrometry (LC/MS/MS) assay. Two hundred picogram per milliliter (pg/mL) of verapamil hydrochloride (Sigma-Aldrich, V4629, CAS 152-11-4) in acetonitrile/methanol, 3/1 by volume, was used as an internal standard and plasma precipitation solvent. To 20 µL of each plasma sample, 100 µL of internal standard solution was added to precipitate matrix proteins. The sample was vortexed then centrifuged with an Eppendorf Centrifuge 5810R (Eppendorf, Hamburg, Germany) at a setting of 4,000 rpm for 5 minutes at 10°C. The supernatant (80 µL) was transferred to a clean 96-well plate and mixed with 75 µL of Milli-Q water. The mixed samples were injected (5 µL) onto a ZORBAX^{®} SB-C8 analytical column (2.1 x 30 mm, 3.5 µm, Agilent Technologies Inc., Palo Alto, CA, USA) using a gradient method at flow rate of 700 µL/min (See Table below). Mobile phases consisting of 0.05% formic acid in water (solvent A) and 0.05% formic acid in acetonitrile (solvent B) were used. Compound A and internal standard were eluted at retention time 1.40 and 1.45 minutes, respectively.

### HPLC Gradient

| **Total Time (min)** | **A (%)** | **B (%)** |
|---|---|---|
| 0.00 | 92 | 8 |
| 2.00 | 10 | 90 |
| 2.01 | 92 | 8 |
| 2.50 | 92 | 8 |

The HPLC system, consisting of Agilent 1260 series binary pump (Agilent Technologies Inc.), Agilent 1260 series micro vacuum degasser (Agilent Technologies Inc.), CTC PAL-HTC-xt analytics autosampler (LEAP Technologies, Carborro, NC, USA) was interfaced to a Applied Biosystems SCIEX triple quadrupole 5500 mass spectrometer (AB Sciex LLC., Foster City, CA, USA). Mass spectral analyses were carried out using electrospray ionization (ESI) in the positive ion mode. Compound A (363.03>156.00) and internal standard (455.40>165.10) peak integration were performed using Analyst^{™} 1.5 software. The lower limit of quantitation (LLOQ) in plasma was 5 pg/mL. Known amounts of Compound A were spiked into plasma to create quality control samples with known concentrations of 20, 80, 800, 4000 and 20000 pg/mL.

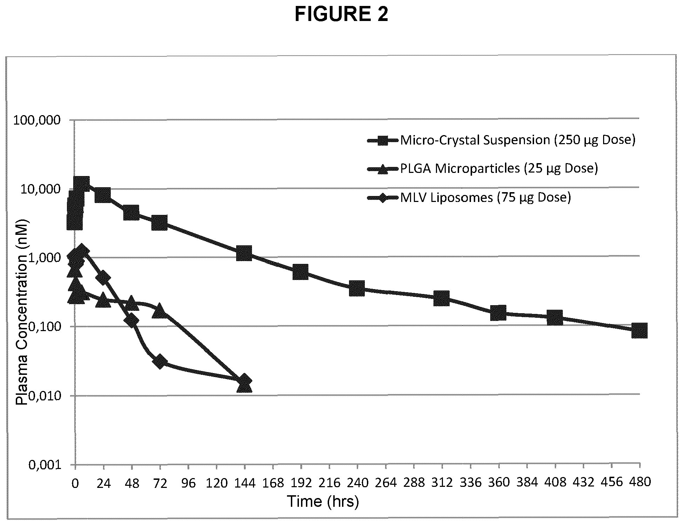

At time points up to 480 hours, rat plasma samples were collected and assayed for drug concentration. Table 9 shows the plasma pK parameters after intra-articular administration. As shown in FIG. 2 and Table 9, the microcrystal suspension outperformed the other formulations in enabling the highest drug dose, highest Cmax and drug exposure over time (AUC) until the end of the observation period. The microsuspension profile (■) reaches the highest Cmax, since the drug load was the highest among the systems tested. Due to the longer residence time of microcrystal suspension of Compound A, the microcrystal suspension of Compound A was further profiled *in vivo.*

**Table 9**

| **Formulation** | **Dose [µg]** | **T_{1/2} [hrs]** | **Cₘₐₓ [nM]** | **Tₘₐₓ [hrs]** | **AUC_{0-inf} [hrs*nM/µ]** | **Dose (normalized) AUC_{0-inf} [hrs*nM/µg]** | **Dose (normalized) Cmax [nM/pg]** |
|---|---|---|---|---|---|---|---|
| Microcrystal suspension | 250 | 102.4 | 11.8 | 6.0 | 761.9 | 3.0 | 0.047 |
| PLGA microparticle suspension | 25 | 43 | 0.74 | 0.69 | 26.4 | 1.1 | 0.029 |
| MLV liposome suspension | 75 | 18.8 | 1.9 | 2.2 | 33.1 | 0.44 | 0.025 |

### Example 8. Immediate and Extended Release Formulation Comparative Data

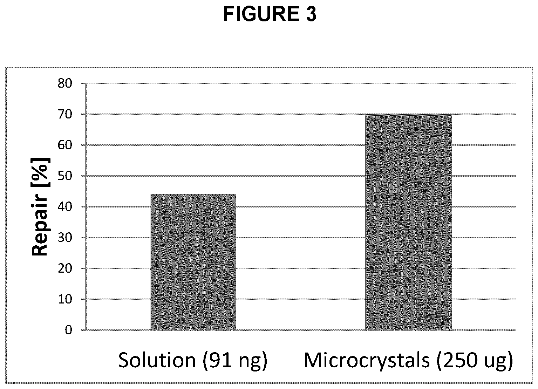

An *in vivo* study compared the immediate release (IR) and extended release (ER) formulations at deliverable dosages achievable with the respective formulation types in a rat meniscal tear (RMT) model in 36-week old Lewis males, following a single intra-articular injection after 4 weeks and take-down after 12 weeks (i.e., 8 weeks after injection). Figure 3 compares the *in vivo* profiles of Compound A ER formulation (250 µg) and IR formulation (91 ng). As shown in Figure 3, the extended release micro-crystal suspension was effective in repairing cartilage, and therefore provides the advantage of reduced dosing frequency (i.e., fewer injections) without compromising effectiveness of treatment.

### Example 9. Extended Release Formulations comprising Compound A (25 mg/mL)

Compound A (25 mg/mL) was dispersed in an aqueous buffered vehicle containing the following excipients, in the presence of anhydrous disodium phosphate (5 mM), NaOH (1N), HCl 25% (1N) as pH adjusting agents to set the pH to physiological pH 7, and water for injection.

| **Vehicle** | **w/v (%)** |
|---|---|
| PVP-K12 | 2% |
| Poloxamer 407 (LUTROL ^{®} F127) | 0.1 % |
| NaCl | .88 % |

To increase the concentration of the drug substance, 50 mg of Compound A powder was added to 2 mL of vehicle solution as above. The solution was stirred overnight at 500 rpm with magnetic stirring, and a homogeneous suspension of individually dispersed microcrystals with no visible aggregates or agglomerates was obtained.

### Example 10. Extended Release Formulation comprising Compound A (50-400 mg/mL)

Compositions comprising essentially the same vehicle composition as in Example 10 with varying concentrations of PVP K12 and poloxamer 407 were screened to evaluate the impact of excipient concentration on sedimentation and re-suspension of compositions comprising Compound A at concentrations of about 50 mg/mL to about 400 mg/mL. The following compositions (1)-(4) were obtained as dis-agglomerated suspensions that were easily resuspended after sedimentation (e.g., during autoclaving or upon storage for several days).

| | (1) | (2) | (3) | (4) |
|---|---|---|---|---|
| Compound A | 50 mg/mL | 100 mg/mL | 200 mg/mL | 400 mg/mL |
| PVP-K12 | 2-4% (w/v) | 2-4% (w/v) | 2-4% (w/v) | 2-4% (w/v) |
| Poloxamer 407 | 0.2% (w/v) | 0.2% (w/v) | 0.2%-0.4% (w/v) | 0.8% (w/v) |

### Example 11. Carboxymethyl Cellulose as Additional Suspension Stabilizer

Carboxymethyl cellulose was added to a suspension comprising Compound A microcrystals (25 mg/mL) in the buffered vehicle of Example 6. Surprisingly, resuspension of non-autoclaved and auto-claved samples were improved in the presence of CMC (1% and 1.5% w/v).

## Claims

1. A pharmaceutical composition comprising an aqueous suspension of: (i) microcrystalline N-(3,4-dichlorophenyl)-3-(pyridin-4-yl)-7-oxabicyclo[2.2.1]heptane-2-carboxamide or a pharmaceutically acceptable salt thereof; and (ii) a surfactant comprising a water-soluble co-polymer **characterized by** > 5% solubility in water at 25 °C.

2. The pharmaceutical composition of claim 1, wherein said surfactant is a water-soluble block co-polymer of formula 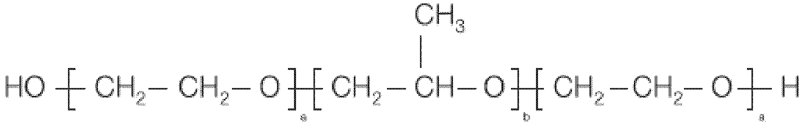 wherein:
a is 75-101; and
b is 25-60.

3. The pharmaceutical composition of claim 2, wherein said water-soluble block co-polymer is further defined as poloxamer 188 or poloxamer 407.

4. The pharmaceutical composition of claims 2 or 3, wherein the concentration of said water-soluble block co-polymer is at least 0.025% w/v, and optionally between 0.05-1% w/v.

5. The pharmaceutical composition of claim 1, wherein said surfactant further comprises sodium lauryl sulfate, optionally wherein the concentration of said sodium lauryl sulfate is at least 0.05 % w/v, and optionally between 0.05-0.5% w/v.

6. The pharmaceutical composition of any one of claims 1-5, further comprising a suspension stabilizer, optionally wherein the concentration of said suspension stabilizer is between 0.1-10% w/v.

7. The pharmaceutical composition of claim 6, wherein said suspension stabilizer is selected from: (i) polyvinylpyrrolidone having an average molecular weight between 1-10 kDa, and optionally between 2-5 kDa; (ii) carboxymethyl cellulose having an average molecular weight between 25-2500 kDa, and optionally between 75-125 kDa; and (iii) a combination thereof.

8. The pharmaceutical composition of claim 7, wherein the concentration of said polyvinylpyrrolidone is between 1-5% w/v, and optionally between about 2-4% w/v; and the concentration of said carboxymethyl cellulose is 0.5-2% (w/v), and optionally between 0.75-1.5% w/v.

9. The pharmaceutical composition of claim 8, wherein said polyvinylpyrrolidone is further defined as PVP-12.

10. The pharmaceutical composition of claim 1, wherein said aqueous suspension comprises: (i) between 1-400 mg of microcrystalline N-(3,4-dichlorophenyl)-3-(pyridin-4-yl)-7-oxabicyclo[2.2.1] heptane-2-carboxamide per 1 mL of said aqueous suspension; (ii) between 0.05-1% w/v of water-soluble block co-polymer, optionally wherein said water-soluble block co-polymer is poloxamer 188 or poloxamer 407; and optionally (iii) between 1-5% w/v of polyvinylpyrrolidone, optionally wherein said polyvinylpyrrolidone is PVP-K12.

11. The pharmaceutical composition of any one of claims 1-10, wherein said N-(3,4-dichlorophenyl)-3-(pyridin-4-yl)-7-oxabicyclo[2.2.1]heptane-2-carboxamide is further defined as (1R,2R,3S,4S)-N-(3,4-dichlorophenyl)-3-(pyridin-4-yl)-7-oxabicyclo[2.2.1] heptane-2-carboxamide.

12. The pharmaceutical composition of any one of claims 1-11, wherein said composition is suitable for intra-articular injection.

13. The pharmaceutical composition of any one of claims 1 to 12 for use in treating, ameliorating or preventing acute joint damage or injury in a subject in need thereof, and optionally in combination with a second therapeutic agent.

14. The pharmaceutical composition for use according to claim 13, wherein said pharmaceutical composition comprises a dose range of up to 25 mg, up to 75 mg, up to 100 mg, up to 200 mg or up to 400 mg, of microcrystalline N-(3,4-dichlorophenyl)-3-(pyridin-4-yl)-7-oxabicyclo[2.2.1]heptane-2-carboxamide.

15. A combination comprising the pharmaceutical composition of any one of claims 1-12, and a second therapeutic agent.

## Patentansprüche

1. Pharmazeutische Zusammensetzung, umfassend eine wässrige Suspensionen von: (i) mikrokristallinem N-(3,4-Dichlorphenyl)-3-(pyridin-4-yl)-7-oxabicyclo[2.2.1]heptan-2-carboxamid oder einem pharmazeutisch unbedenklichen Salz davon und (ii) einem Tensid, das ein wasserlösliches Copolymer umfasst, das durch > 5 % Löslichkeit in Wasser bei 25 °C gekennzeichnet ist.

2. Pharmazeutische Zusammensetzung nach Anspruch 1, wobei es sich bei dem Tensid um ein wasserlösliches Blockcopolymer der Formel 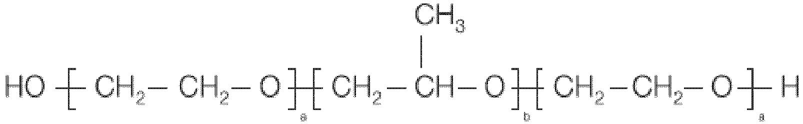 handelt, wobei:
a für 75-101 steht und
b für 25-60 steht.

3. Pharmazeutische Zusammensetzung nach Anspruch 2, wobei das wasserlösliche Blockcopolymer ferner als Poloxamer 188 oder Poloxamer 407 definiert ist.

4. Pharmazeutische Zusammensetzung nach Anspruch 2 oder 3, wobei die Konzentration des wasserlöslichen Blockcopolymers mindestens 0,025 % w/v beträgt und gegebenenfalls zwischen 0,05-1 % w/v liegt.

5. Pharmazeutische Zusammensetzung nach Anspruch 1, wobei das Tensid ferner Natriumlaurylsulfat umfasst, gegebenenfalls wobei die Konzentration des Natriumlaurylsulfats mindestens 0,05 % w/v beträgt und gegebenenfalls zwischen 0,05-0,5 % w/v liegt.

6. Pharmazeutische Zusammensetzung nach einem der Ansprüche 1-5, ferner umfassend einen Suspensionsstabilisator, gegebenenfalls wobei die Konzentration des Suspensionsstabilisators zwischen 0,1-10 % w/v liegt.

7. Pharmazeutische Zusammensetzung nach Anspruch 6, wobei der Suspensionsstabilisator aus (i) Polyvinylpyrrolidon mit einem mittleren Molekulargewicht zwischen 1-10 kDa und gegebenenfalls zwischen 2-5 kDa; (ii) Carboxymethylcellulose mit einem mittleren Molekulargewicht zwischen 25-2500 kDa und gegebenenfalls zwischen 75-125 kDa und (iii) einer Kombination davon ausgewählt ist.

8. Pharmazeutische Zusammensetzung nach Anspruch 7, wobei die Konzentration des Polyvinylpyrrolidons zwischen 1-5 % w/v und gegebenenfalls zwischen etwa 2-4 % w/v liegt und die Konzentration der Carboxymethylcellulose 0,5-2 % (w/v) beträgt und gegebenenfalls zwischen 0,75-1,5 % w/v liegt.

9. Pharmazeutische Zusammensetzung nach Anspruch 8, wobei das Polyvinylpyrrolidon ferner als PVP-12 definiert ist.

10. Pharmazeutische Zusammensetzung nach Anspruch 1, wobei die wässrige Suspension Folgendes umfasst: (i) zwischen 1-400 mg mikrokristallines N-(3,4-Dichlorphenyl)-3-(pyridin-4-yl)-7-oxabicyclo[2.2.1]heptan-2-carboxamid pro 1 ml der wässrigen Suspension; (ii) zwischen 0,05-1 % w/v wasserlösliches Blockcopolymer, gegebenenfalls wobei es sich bei dem wasserlöslichen Blockcopolymer um Poloxamer 188 oder Poloxamer 407 handelt; und gegebenenfalls (iii) zwischen 1-5 % w/v Polyvinylpyrrolidon, gegebenenfalls wobei es sich bei dem Polyvinylpyrrolidon um PVP-K12 handelt.

11. Pharmazeutische Zusammensetzung nach einem der Ansprüche 1-10, wobei das N-(3,4-Dichlorphenyl)-3-(pyridin-4-yl)-7-oxabicyclo[2.2.1]heptan-2-carboxamid ferner als (1R,2R,3S,4S)-N-(3,4-Dichlorphenyl)-3-(pyridin-4-yl)-7-oxabicyclo[2.2.1]heptan-2-carboxamid definiert ist.

12. Pharmazeutische Zusammensetzung nach einem der Ansprüche 1-11, wobei die Zusammensetzung für die intraartikuläre Injektion geeignet ist.

13. Pharmazeutische Zusammensetzung nach einem der Ansprüche 1 bis 12 zur Verwendung bei der Behandlung, Linderung oder Prävention von akuter Gelenkschädigung oder -verletzung bei einem Individuum, bei dem diesbezüglicher Bedarf besteht, und gegebenenfalls in Kombination mit einem zweiten therapeutischen Mittel.

14. Pharmazeutische Zusammensetzung zur Verwendung nach Anspruch 13, wobei die pharmazeutische Zusammensetzung einen Dosisbereich von bis zu 25 mg, bis zu 75 mg, bis zu 100 mg, bis zu 200 mg oder bis zu 400 mg mikrokristallinem N-(3,4-Dichlorphenyl)-3-(pyridin-4-yl)-7-oxabicyclo[2.2.1]heptan-2-carboxamid umfasst.

15. Kombination, umfassend die pharmazeutische Zusammensetzung nach einem der Ansprüche 1-12 und ein zweites therapeutisches Mittel.

## Revendications

1. Composition pharmaceutique comprenant une suspension aqueuse de : (i) du *N*-(3,4-dichlorophényl)-3-(pyridin-4-yl)-7-oxabicyclo[2.2.1]heptane-2-carboxamide microcristallin ou un sel pharmaceutiquement acceptable de celui-ci ; et (ii) un tensioactif comprenant un copolymère hydrosoluble **caractérisé par** une solubilité > 5 % dans l'eau à 25 °C.

2. Composition pharmaceutique selon la revendication 1, dans laquelle ledit tensioactif est un copolymère à blocs hydrosoluble de formule 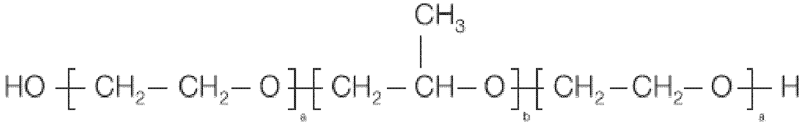 dans laquelle :
a vaut 75 à 101 ; et
b vaut 25 à 60.

3. Composition pharmaceutique selon la revendication 2, dans laquelle ledit copolymère à blocs hydrosoluble est en outre défini comme le poloxamère 188 ou le poloxamère 407.

4. Composition pharmaceutique selon les revendications 2 ou 3, dans laquelle la concentration dudit copolymère à blocs hydrosoluble est d'au moins 0,025 % p/v et éventuellement comprise entre 0,05 et 1 % p/v.

5. Composition pharmaceutique selon la revendication 1, dans laquelle ledit tensioactif comprend en outre du laurylsulfate de sodium, éventuellement dans laquelle la concentration dudit laurylsulfate de sodium est d'au moins 0,05 % p/v et éventuellement comprise entre 0,05 et 0,5 % p/v.

6. Composition pharmaceutique selon l'une quelconque des revendications 1 à 5, comprenant en outre un stabilisant de suspension, éventuellement dans laquelle la concentration dudit stabilisant de suspension est comprise entre 0,1 et 10 % p/v.

7. Composition pharmaceutique selon la revendication 6, dans laquelle ledit stabilisant de suspension est choisi parmi : (i) de la polyvinylpyrrolidone ayant une masse moléculaire moyenne comprise entre 1 et 10 kDa et éventuellement entre 2 et 5 kDa ; (ii) de la carboxyméthylcellulose ayant une masse moléculaire moyenne comprise entre 25 et 2500 kDa et éventuellement entre 75 et 125 kDa ; et (iii) une combinaison de celles-ci.

8. Composition pharmaceutique selon la revendication 7, dans laquelle la concentration de ladite polyvinylpyrrolidone est comprise entre 1 et 5 % p/v et éventuellement entre environ 2 et 4 % p/v ; et la concentration de ladite carboxyméthylcellulose est de 0,5 à 2 % (p/v) et éventuellement comprise entre 0,75 et 1,5 % p/v.

9. Composition pharmaceutique selon la revendication 8, dans laquelle ladite polyvinylpyrrolidone est en outre définie comme la PVP-12.

10. Composition pharmaceutique selon la revendication 1, dans laquelle ladite suspension aqueuse comprend : (i) entre 1 et 400 mg de *N*-(3,4-dichlorophényl)-3-(pyridin-4-yl)-7-oxabicyclo[2.2.1]heptane-2-carboxamide microcristallin pour 1 ml de ladite suspension aqueuse ; (ii) entre 0,05 et 1 % p/v de copolymère à blocs hydrosoluble, éventuellement ledit copolymère à blocs hydrosoluble étant le poloxamère 188 ou le poloxamère 407 ; et éventuellement (iii) entre 1 et 5 % p/v de polyvinylpyrrolidone, éventuellement ladite polyvinylpyrrolidone étant la PVP-K12.

11. Composition pharmaceutique selon l'une quelconque des revendications 1 à 10, dans laquelle ledit N-(3,4-dichlorophényl)-3-(pyridin-4-yl)-7-oxabicyclo[2.2.1]heptane-2-carboxamide est en outre défini comme le (1*R*, 2*R*, 3*S*, 4*S*)-*N*-(3,4-dichlorophényl)-3-(pyridin-4-yl)-7-oxabicyclo[2.2.1]heptane-2-carboxamide.

12. Composition pharmaceutique selon l'une quelconque des revendications 1 à 11, ladite composition étant appropriée pour une injection intra-articulaire.

13. Composition pharmaceutique selon l'une quelconque des revendications 1 à 12 destinée à être utilisée en traitement, amélioration ou prévention d'atteinte ou lésion articulaire aiguë chez un sujet qui en a besoin et éventuellement en combinaison avec un second agent thérapeutique.

14. Composition pharmaceutique destinée à être utilisée selon la revendication 13, ladite composition pharmaceutique comprenant une plage de dose allant jusqu'à 25 mg, jusqu'à 75 mg, jusqu'à 100 mg, jusqu'à 200 mg ou jusqu'à 400 mg de *N*-(3,4-dichlorophényl)-3-(pyridin-4-yl)-7-oxabicyclo[2.2.1]heptane-2-carboxamide microcristallin.

15. Combinaison comprenant la composition pharmaceutique selon l'une quelconque des revendications 1 à 12 et un second agent thérapeutique.
